# EUROPÄISCHE PATENTSCHRIFT

(11) **EP 1 003 705 B1**
(45) Veröffentlichungstag und Bekanntmachung des Hinweises auf die Patenterteilung: **25.06.2003**
(21) Anmeldenummer: 98942620.0
(22) Anmeldetag: 24.07.1998
(51) Int. Cl.: C07C 45/89, C07C 49/90

(54) **EINSATZ VON PHOSPHORSÄURE ALS HOMOGENER KATALYSATOR BEI DER HERSTELLUNG VON KETEN**
USE OF PHOSPHORIC ACID AS HOMOGENEOUS CATALYST DURING THE PREPARATION OF KETENE
UTILISATION D'ACIDE PHOSPHORIQUE COMME CATALYSEUR HOMOGENE LORS DE LA PREPARATION DE CETENE

(30) Priorität: 07.08.1997 DE 19734275
(43) Veröffentlichungstag der Anmeldung: 31.05.2000
(73) Patentinhaber: Clariant GmbH, 65929 Frankfurt am Main (DE)
(72) Erfinder: BAURMEISTER, Jochen, D-60529 Frankfurt (DE); SCHÄFER, Thomas, D-64646 Heppenheim (DE)
(86) Internationale Anmeldenummer: EP9804654
(87) Internationale Veröffentlichungsnummer: WO99007662

(56) Entgegenhaltungen:
- DE-A- 2 059 292
- GB-A- 478 303
- US-A- 2 278 537
- US-A- 2 856 426
- US-A- 3 378 583

## Beschreibung

Die Erfindung betrifft ein Verfahren zur homogen katalysierten Essigsäurepyrolyse zur Herstellung von Keten und/oder Folgeprodukten.

Die homogen katalysierte Pynolyse von Essigsäure wird zur Produktion von Essigsäureanhydrid, Diketen und Keten im industriellen Maßstab durchgeführt. Keten wird direkt zu Folgeprodukten, z.B. Sorbinsäure und Dimethylacrylsäuretacton weiterverarbeitet. Die Reaktion läuft bei höheren Temperaturen im Bereich von 400 bis 800 °C und bei Unterdruck ab. Neben den Hauptprodukten Wasser und Keten werden unter den genannten Reaktionsbedingungen eine Reihe von Nebenprodukten, z.B. Kohlenmonoxid, Kohlendioxid, Ethen, Ethin, Methan, Propadien und Wasserstoff gebildet. Nach der Reaktion werden im weiteren Prozeßablauf nicht umgesetzte Essigsäure und Wasser auskondensiert und die Prozeßgase mit Essigsäureanhydrid gewaschen. Die folgenden Verfahrensschritte hängen vom Zielprodukt ab.

Bislang werden im industriellen Maßstab nur Phosphorsäurederivate mit einem definierten Dampfdruck, z.B. flüssiges Triethylphosphat mit einem Siedepunkt von 215 °C zur Katalyse des Prozesses eingesetzt. Diese sind jedoch vergleichsweise teuer. Es ist zwar aus der DE 687 065 bekannt, als Katalysator Phosphorsäure einzusetzen, jedoch nur unter den Bedingungen einer heterogenen Katalyse und des Labormaßstabs.

GB-A-478 303 beschreibt ein Verfahren zur Herstellung von Keten, Essigsäureanhydrid oder deren Homologa durch thermische Dehydratation von Essigsäure in Gegenwart von Phosphorsäure. Die Zugabe der Phosphorsäure erfolgt hierbei als Flüssigkeit, wobei die flüssige Lösung an der Verbindungsstelle der zwei Arme des Y-Stückes auf den überhitzten Essigsäuredampf trifft und dort verdampft.

Die in der älteren Patentliteratur vorgeschlagenen heterogenen Verfahren konnten sich in der industriellen Praxis nicht durchsetzten, da die Ketenausbeuten im Vergleich zur homogenen Katalyse z.B. mit Triethylphosphat deutlich geringer sind und Katalysatorschüttungen von Phosphorsäurederivaten und Phosphaten unter den Reaktionsbedingungen durch die Belegung mit Koks schnell deaktiviert und zu einer Verstopfung des Reaktors führten.

Der Erfindung lag daher die Aufgabe zugrunde, das eingangs genannte Verfahren dahingehend zu verbessern billigere homogene Katalysatoren mit gleicher oder besserer katatytischer Wirkung wie Triethytphoshat zu identifizieren.

Es wurde nun überraschenderweise gefunden, daß im Vergleich zu Triethylphosphat die gleiche Ketenselektivität und gleicher Essigsäureumsatz erhalten werden kann, wenn man als Katalysator Phosphorsäure in flüssiger Form in den Essigsäuredampf verdüst.
Dies widerspricht bisherigen Untersuchungen und Literaturangaben, nach denen die katalytische Aktivität von Triethylphosphat wesentlich besser sein soll und überrascht um so mehr, da Phosphorsäure als schwer verdampfbar gilt, keinen definierten Siedepunkt besitzt und beim Verdampfen unter Wasseraustritt höhermolekulare Kondensationsprodukte gebildet werden. Man erwartet, daß die bei hoher Temperatur entstehenden Kondensationsprodukte der Phosphorsäure (z.B. Metaphosphorsäure und Phosphoroxide) die Anlagen verstopfen.

Gegenstand der Erfindung ist daher ein Verfahren zur homogen katalysierten Essigsäurepyrolyse zur Herstellung von Keten und/oder Folgeprodukten, dadurch gekennzeichnet, daß man in den Essigsäuredampf als Katalysator Phosphorsäure in Form eines Flüssigkeitsstrahls verdüst.

Der Begriff Phosphorsäure umfaßt im Sinne der Erfindung sämtliche Sauerstoffsäuren des Phosphors und Lösungen deren Anhydride, jeweils einzeln oder in Mischung. Bevorzugt sind Orthophosphorsäure, Lösungen von Metaphosphorsäuren, Oligomeren der Phosphorsäure und Phosphorpentoxid.
Der molare Anteil der Phosphorsäure beträgt vorzugsweise 100 bis 10000 ppm berechnet als molarer Anteil an elementarem Phosphor (P) bezogen auf die insgesamt eingesetzte Stoffmenge an reiner Essigsäure (CH₃COOH).

Besondere Ausführungsformen ergeben sich aus den Unteransprüchen.

Die Phosphorsäure wird vorzugsweise als handelsübliche Phosphorsäure (75, 80 oder 85 %) oder verdünnt bei höherer Temperatur flüssig in den Essigsäuredampf verdüst. Sie kann mit anorganischen oder organischen Lösungsmitteln verdünnt zugegeben werden, bevorzugt mit Wasser, Methanol, Ethanol, Essigsäureanhydrid oder Aceton.

In einer bevorzugten Ausführungsform wird die Phosphorsäure mit Wasser oder Essigsäure im Verhältnis bis höchstens 1:100 verdünnt und bei Temperaturen über 400 °C als kontinuierlicher Flüssigkeitsstrahl mit hohem Impuls im Essigsäurehauptstrom verdüst. Das bedeutet, daß die Geschwindigkeit des Flüssigkeitsstrahls mindestens 10 mal höher ist als die des Essigsäurehauptstromes. Dabei wird ein Wandkontakt des Flüssigkeitsstrahls vermieden. Durch die hohe Temperatur und gute Durchmischung mit der vorgewärmten Essigsäure gelingt es, die Phophorsäure schnellstmöglich und nahezu vollständig zu verdampfen. Unter diesen Bedingungen wurde die gleiche katalytische Aktivität von Phosphorsäure und Triethylphosphat festgestellt. Feste Ablagerungen im Bereich der Katalysatorzumischung und im Reaktor traten dabei nicht auf.

Weitere bevorzugte Ausführungsformen sind dadurch gekennzeichnet, daß die Zugabe des Katalysators mit Gaszusatz, bevorzugt mit Stickstoff oder Ammoniak erfolgt und/oder daß die Zugabe über Düsen, vorzugsweise über Ultraschalldüsen oder Zweistoffdüsen erfolgt.

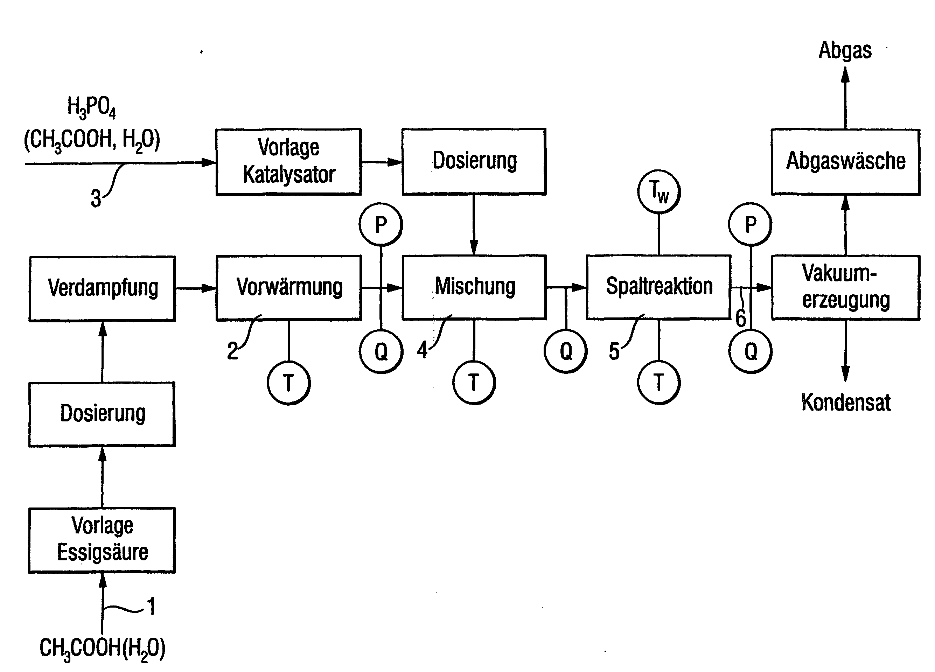

Im folgenden wird eine beispielhafte Ausführungsform der Erfindung anhand des in der Figur dargestellten vereinfachten Fließbildes näher beschrieben. Eine Beschränkung der Erfindung in irgendeiner Weise ist dadurch nicht beabsichtigt.

Gemäß der Figur wird Essigsäure 1 in einem Vorwärmer 2 aufgeheizt (600°C), anschließend mit einem Katalysator 3, versetzt, in einem beheizten Mischer 4 mit dem Katalysator 3, der flüssig zudosiert wird, intensiv vermischt und in beheizten Reaktor 5 zur Reaktion gebracht. Das entstehende Reaktionsgemisch 6 wird aus dem Reaktor 5 abgezogen. Vor dem Mischer 4, vor dem Reaktor 5 und nach dem Reaktor 5 werden aus den Prozeßgasströmen mittels Pumpen Proben abgezogen und einem Massenspektrometer zur Analyse zugeführt. Die Steuerung erfolgt durch ein nicht dargestelltes Prozeßleitsystem.

Die folgenden Versuche dienen ebenfalls der näheren Erläuterung der Erfindung, ohne diese dadurch zu beschränken.

Eine Phosphorsäurelösung in Wasser und Essigsäure wurde in einer solchen Versuchsapparatur bei 580 °C und einem Druck von 300 mbar einem vorgewärmtem Essigsäurestrom als kontinuierlicher Flüssigkeitsstrahl zugemischt. Offenbar ist die Temperatur hoch genug, daß die Phosphorsäure (PS) unter Bildung der katalytisch aktiven Spezies vollständig in die Gasphase übergeht. Die Umsetzung in einem Rohrrektor bei 300 mbar, mittleren Verweilzeiten von 0,5 bis 5 s und Austrittstemperaturen von 650 bis 750 °C führte jeweils zum gleichen Produktspektrum, das man aus Vergleichsversuchen mit Triethylphosphat (TEP) als Katalysator erhält.

Die Versuchsbedingungen und Ergebnisse einiger ausgewählter Versuche sind in der nachstehenden Tabelle zusammengefaßt.

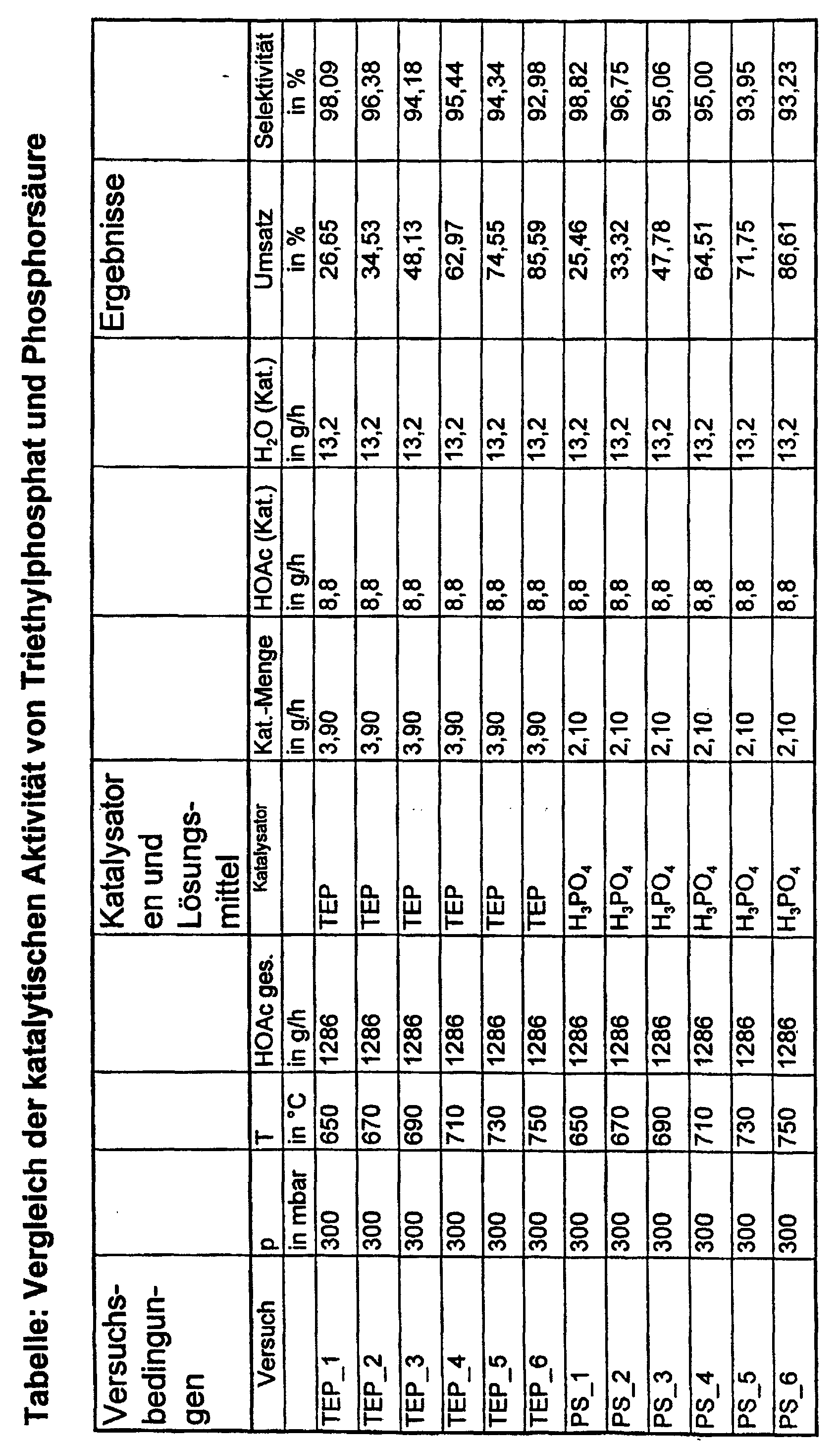

Die lineare Geschwindigkeit des Flüssigkeitsstrahls betrug bei den in der Tabelle genannten Technikumsyersuchen etwa 100 m/s am Düsenaustritt. Dieser Wert wurde aus dem Durchmesser des eingesetzten Düsenplättchens und einem Durchsatz von 20 ml/h (25 g /h) Katalysatorlösung berechnet. Die lineare Geschwindigkeit der verdampften Essigsäure (1200 g/h, 600 °C) betrug im Bereich der Katalysatorverdüsung (Rohrduchmesser 20 mm) etwa 4 m/s.

Bei den durchgeführten Versuchen wurde der Katalysator jeweils als Lösung in Wasser und Essigsäure als kontinuierlicher Flüssigkeitsstrahl dem Hautstrom zugemischt. Der über die Katalysatordüse zugemischte Essigsäure- bzw. Wasseranteil betrug bezogen auf den vorgewärmten Essigsäurehauptstrom jeweils etwa 0,7 bzw. 3,4 mol-%.

Der Katalysatoranteil bezogen auf die gesamt eingesetzte Essigsäuremenge in den Versuchen (Hauptstrom und Zumischung in einer Katalysatorvorlage) betrug jeweils 1000 ppm.

Überraschenderweise wurde bei diesen Messungen laut Tabelle eine lange Betriebsdauer der Apparatur beim Einsatz von Phosphorsäure und, im Rahmen der Meßgenauigkeit, gleiche Selektivität und gleicher Umsatz bezogen auf den Vergleichsversuch mit TEP nachgewiesen. Eine vermutete schnelle Verstopfung des Rohrreaktors durch feste Zersetzungsprodukte der Phosphorsäure (z.B. P₂O₅) wurde nicht beobachtet, wahrscheinlich aufgrund der hohen Temperatur der Katalysator-Mischkonstruktion und/oder der guten Durchmischung und/oder der Verdünnung des Katalysators.

Die Vorteile des erfindungsgemäßen Verfahrens sind im wesentlichen darin zu sehen, daß Triethylphosphat und andere Katalysatoren bei optimierten Eindüsungsbedingungen bei gleicher Selektivität und Umsatz durch Phosphorsäure ersetzt werden können Damit ergibt sich eine großes Einsparpotential bei der industriellen Herstellung von Keten und dessen Folgeprodukten.

## Patentansprüche

1. Verfahren zur katalysierten Essigsäurepyrolyse zur Herstellung von Keten und/oder Folgeprodukten, **dadurch gekennzeichnet, daß** man in den Essigsäuredampf als Katalysator eine Sauerstoffsäure des Phosphors oder eine Lösung des Anhydrids der Säure einzeln oder in Mischung in Form eines Flüssigkeitsstrahls verdüst, wobei die Geschwindigkeit des Flüssigkeitsstrahls mindestens 10 mal höher ist als die des Essigsäuredampfes und der Katalysator mit Wasser oder Essigsäure im Verhältnis bis höchstens 1:100 verdünnt und bei Temperaturen über 400 °C im Essigsäurehauptstrom verdüst wird.

2. Verfahren nach Anspruch 1, **dadurch gekennzeichnet, daß** man den Katalysator mit anorganischen oder organischen Lösungsmitteln verdünnt verdüst, bevorzugt mit Wasser, Methanol, Ethanol oder Aceton.

3. Verfahren nach mindestens einem der Ansprüche 1 oder 2, **dadurch gekennzeichnet, daß** die Zugabe des Katalysators mit Gaszusatz, bevorzugt mit Stickstoff oder Ammoniak erfolgt.

4. Verfahren nach mindestens einem der Ansprüche 1 bis 3, **dadurch gekennzeichnet, daß** die Zugabe über Düsen, vorzugsweise über Ultraschalldüsen oder Zweistoffdüsen erfolgt.

## Claims

1. Process for the catalyzed pyrolysis of acetic acid for the preparation of ketene and/or secondary products, **characterized in that**, as catalyst, an oxygen acid of phosphorus or a solution of the anhydride of the acid is atomized into the acetic acid vapour, individually or as a mixture, in the form of a liquid jet, where the velocity of the liquid jet is at least 10 times higher than that of the acetic acid vapour and the catalyst is diluted with water or acetic acid in a maximum ratio of 1:100 and is atomized in the main acetic acid stream at temperatures above 400°C.

2. Process according to claim 1, **characterized in that** the catalyst is atomized diluted with inorganic or organic solvents, preferably with water, methanol, ethanol or acetone.

3. Process according to at least one of Claims 1 or 2, **characterized in that** the catalyst is added with addition of gas, preferably with nitrogen or ammonia.

4. Process according to at least one of Claims 1 to 3, **characterized in that** the addition takes place via nozzles, preferably via ultrasound nozzles or two-component nozzles.

## Revendications

1. Procédé destiné à la pyrolyse de l'acide acétique catalysée pour la fabrication de cétènes et/ou de produits réactionnels, **caractérisé en ce que** l'on atomise dans la vapeur d'acide acétique comme catalyseur un oxoacide du phosphore ou une solution de l'anhydride acétique séparément ou en mélange, sous forme d'un jet de liquide, la vitesse du jet de liquide étant au moins dix fois plus élevée que celle de la vapeur de l'acide acétique et le catalyseur étant dilué dans de l'eau ou dans de l'acide acétique dans un rapport de 1 à 100 au plus et étant atomisé à des températures de plus de 400 °C dans le flux principal d'acide acétique.

2. Procédé selon la revendication 1, **caractérisé en ce que** l'on atomise le catalyseur dilué avec des solvants inorganiques ou organiques, de préférence avec de l'eau, du méthanol, de l'éthanol ou de l'acétone.

3. Procédé selon au moins l'une des revendications 1 ou 2, **caractérisé en ce que** l'addition du catalyseur se produit avec un ajout de gaz, de préférence avec de l'azote ou de l'ammoniac.

4. Procédé selon au moins l'une des revendications 1 à 3, **caractérisé en ce que** le rajout au moyen des buses se produit, de préférence, par des buses à ultrason ou des buses binaires.
